# EUROPEAN PATENT APPLICATION

(11) **EP 3 135 340 A1**
(43) Date of publication of application: **01.03.2017**
(21) Application number: 16186092.9
(22) Date of filing: 29.08.2016
(51) Int. Cl.: A61Q 3/02, A61K 8/81, C08K 5/5397

(54) **GEL NAIL POLISH AND MANUFACTURING AND A METHOD OF USING THE NAIL POLISH**

(30) Priority: 24.08.2016 US 201515246520; 29.08.2015 US 201514839926
(71) Applicant: Zhen, Lijuan, City of Industry 91745 (US)
(72) Inventor: Zhen, Lijuan, City of Industry 91745 (US)
(74) Representative: Klunker . Schmitt-Nilson . Hirsch

(57) **Abstract**

A gel nail polish composition includes a gel nail polish mixture and a color agent mixing therewith for forming a gel nail polish layer on a nail of a user. The gel nail polish mixture includes first through fourth chemical elements, wherein the first through fourth chemical elements are aliphatic urethane acrylate, polymer acrylate oligomer, propoxylated neopentyl glycol diacrylate, and trimethylbenzoyl diphenylphosphine oxide. The wipe off base coat layer, the gel nail polish layer, and the top coat layer are dried under an exposure of one of LED light, UV light, and sunlight that no wet sticky colloid is formed after the top coat layer is dried. The gel nail polish layer is adapted for being removed by directly wiping off the gel nail polish layer on the nail by using a rag, cotton, or tissue paper with the nail polish remover.

## Description

### CROSS REFERENCE OF RELATED APPLICATION

This is a continuation application that claims the benefit of priority under 35U.S.C.§119 to a non-provisional application, application number 14/839,926, filed August 29, 2015.

### NOTICE OF COPYRIGHT

A portion of the disclosure of this patent document contains material which is subject to copyright protection. The copyright owner has no objection to any reproduction by anyone of the patent disclosure, as it appears in the United States Patent and Trademark Office patent files or records, but otherwise reserves all copyright rights whatsoever.

### BACKGROUND OF THE PRESENT INVENTION

### FIELD OF INVENTION

The present invention relates to a gel nail polish, and more particularly to an easily wipeable gel nail polish and its manufacturing and using method, wherein the gel nail polish is dried out easily and can be directly wipe off by the polish remover without a complicate removing process.

### DESCRIPTION OF RELATED ARTS

Gel nail polish is relative new in the market but has become really popular at nail salons and for nail painting enthusiasts in a short time. One of the popular gel nail polishes is soft gel polish, which is often called as a soak off gel polish. The current soak off gel polish is long-lasting after applying on the nails, and always looks as new, wherein the soak off gel nails last on the nails for about 2 to 3 weeks without chipping, and they always look like they are just done. In addition, the soak off gel polish can be easily and directly painted on the natural nails in order to do the nail extension, and the user can easily change the color of the soak off gel nails by directly applying the traditional nail polish on the soak off gel nails.

Accordingly, the users need to initially apply the soak off base coat nail polish on their nails as a first layer, and then apply at least one layer of soak off gel nail polish thereon as a second or third layer. Accordingly, the users need to apply a top coat nail polish on the third or fourth layer. In particular, in order to dry each layer on the nail, the nail must be exposed under a LED light or a UV light. In other words, each layer cannot be dried under the sunlight, so that it is inconvenience and money-wasting for the users to prepare and purchase the LED light or the UV light device. In addition, after each layer is dried, some wet sticky colloids will residue on the layer, so that the users must use alcohol to remove wet sticky colloids on each layer and to polish each layer after the following layer is applied. Therefore, the users need to spend at least 60 minutes to finish the four layers soak off gel polish structure, including the soak off gel nail polish applying time, drying time, and residue removing time.

Furthermore, the removing process of the soak off gel nails is inconvenience and troublesome. Since the structure of the soak off gel polish layer is hard (having at least three-layered structure), the user's soak off gel nails cannot be directly wiped off by using a rag, cotton, or tissue paper with the nail polish remover, so the user needs to saturate a piece of cotton ball in the nail polish remover and place it on each of the nails until the cotton makes contact with the entire nail surface, and uses the aluminum foil to wrap around the fingertips until the cotton balls firmly attach on the nails. Waiting for 5 to 7 minutes, a gap will naturally generate between each of the nails and the soak off gel polish layer thereof, and then the user can use cuticle pusher or iron pusher to pop the soak off gel polish layer off the nail. Accordingly, after the soak off gel polish layer is removed from each of the nails, some residue gel is residued on the nail, so that the user must use the nail file or nail buffer to completely remove the residue gel from the nail. For such removing process, the user has to spend at least 30 minutes to remove the soak off nail polish layer from their nails, and the nails will be damaged and thinned by frequently using the nail file or nail buffer.

### SUMMARY OF THE PRESENT INVENTION

The invention is advantageous in that it provides a gel nail polish, wherein the gel nail polish is able to be directly wiped off from the nails by using a rag, cotton, or tissue paper with the nail polish remover without complicated removing process.

Another advantage of the invention is to provide a gel nail polish, wherein the removing process of the gel nail polish of the present invention is simple, so that no aluminum foils are needed to wrap around the fingertips, and also no additional auxiliary, such as a cuticle pusher or iron pusher, is needed to pop the gel nail polish layer off the nail.

Another advantage of the invention is to provide a gel nail polish, wherein no wet sticky colloids will residue on the finished gel nail polish structure after the finished gel nail polish structure is dried, so that the users doesn't need to use alcohol to remove wet sticky colloids on the structure.

Another advantage of the invention is to provide a gel nail polish, wherein the gel nail polish can be easily and quickly dried after exposing under a light structure, wherein, especially, the light structure can be the natural sunlight.

Another advantage of the invention is to provide a method of using a gel nail polish, including applying and removing processes of the gel nail polish, which is timesaving and money saving for the user. In other words, the damages for the nails are reduced during the gel nail polish removing process.

Another advantage of the invention is to provide a gel nail polish, wherein no expensive and complicated structure is required to be employed in the present invention in order to achieve the above mentioned advantages. Therefore, the present invention successfully provides an economic and efficient solution to simplify the applying process and removing process of the gel nail polish.

Additional advantages and features of the invention will become apparent from the description which follows, and may be realized by means of the instrumentalities and combinations particular point out in the appended claims.

According to the present invention, the foregoing and other objects and advantages are attained by a gel nail polish composition which comprises a gel nail polish mixture and a color agent mixing with the gel nail polish mixture.

The gel nail polish mixture comprises first through fourth chemical elements, wherein the first through fourth chemical elements are aliphatic urethane acrylate, polymer acrylate oligomer, propoxylated neopentyl glycol diacrylate which is a monomer as diluting agent, and trimethylbenzoyl diphenylphosphine oxide which is a photo initiator.

In accordance with another aspect of the invention, the present invention comprises a method of manufacturing a gel nail polish, comprising the following steps:
(1) Provide first through fourth chemical elements.
(2) Dissolve the first chemical element and the second chemical element into the third chemical element to form a first mixture.
(3) Mix and stir the fourth chemical element into the first mixture to form a second mixture.
(4) Add and mix the color agent into the second mixture to form a final product of the gel nail polish.
(5) Fill the final product of the gel nail polish into a gel nail polish bottle and package the gel nail polish bottle with the gel nail polish therein, such that the gel nail polish is ready to use.

In accordance with another aspect of the invention, the present invention comprises a method of using a gel nail polish, comprising the following steps:
(A) Apply a wipe off base coat on the nail.
(B) Dry the wipe off base coat under an exposure of light to from a wipe off base coat layer.
(C) Apply a color coat of gel nail polish on the wipe off base coat layer.
(D) Dry the color coat of gel nail polish to form a gel nail polish layer.
(E) Apply a coat of gel nail polish top coat on the gel nail polish layer to from a top coat layer.
(F) Dry the top coat layer to from a finished gel nail polish structure, wherein when the top coat layer is dried, no wet sticky colloid is formed on the finished gel nail polish structure.
(G) To remove the finished gel nail polish structure, directly wipe off the finished gel nail polish structure by a rag, cotton, or tissue paper with the nail polish remover.

In accordance with another aspect of the invention, the present invention comprises a method of using a gel nail polish, comprising the following steps:
(a) Apply a color coat of gel nail polish on the nail.
(b) Dry the color coat of gel nail polish to form a gel nail polish layer.
(c) To remove the gel nail polish layer, directly wipe off the gel nail polish layer by a rag, cotton, or tissue paper with the nail polish remover.

Still further objects and advantages will become apparent from a consideration of the ensuing description and drawings.

These and other objectives, features, and advantages of the present invention will become apparent from the following detailed description, the accompanying drawings, and the appended claims.

### BRIEF DESCRIPTION OF THE DRAWINGS

Fig. 1 is a block diagram illustrating a method of manufacturing a gel nail polish according to a preferred embodiment of the present invention.
Fig. 2 is a block diagram of a method of using a gel nail polish according to the above preferred embodiment of the present invention.
Fig. 3 is a block diagram of an alternative mode of using a gel nail polish according to the above preferred embodiment of the present invention.

### DETAILED DESCRIPTION OF THE PREFERRED EMBODIMENT

The following description is disclosed to enable any person skilled in the art to make and use the present invention. Preferred embodiments are provided in the following description only as examples and modifications will be apparent to those skilled in the art. The general principles defined in the following description would be applied to other embodiments, alternatives, modifications, equivalents, and applications without departing from the spirit and scope of the present invention.

A gel nail polish of the present invention is adapted to being dried under an exposure of light. The user is able to remove the gel nail polish structure by directly wiping off the gel nail polish structure by a rag, cotton, or tissue paper soaked with the nail polish remover without complicated removing process and auxiliary tools as the conventional nail polish removing process.

The gel nail polish according to the preferred embodiment of the present invention is illustrated, wherein the gel nail polish composition comprises a gel nail polish mixture and a color agent mixing with the nail polish mixture. The gel nail polish mixture comprises first through fourth chemical elements, wherein the first through fourth chemical elements are aliphatic urethane acrylate, polymer acrylate oligomer, propoxylated neopentyl glycol diacrylate which is a monomer as diluting agent, and trimethylbenzoyl diphenylphosphine oxide which is a photo initiator respectively.

Accordingly, an amount of the first chemical element (aliphatic urethane acrylate) has a range of 57.6-69.9% by weight. An amount of the second chemcial element (polymer acrylate oligomer) has a range of 16.4-19.8% by weight. An amount of the third chemcial element (dilulting agent embodied as propoxylated neopentyl glycol diacrylate) has a range of 12.7-15.4% by weight. An amount of the fourth chemcial element (photo initiator embodied as trimethylbenzoyl diphenylphosphine oxide) has a range of 4.1-4.9% by weight. In particular, a weight ratio of the first through fourth chemcial element is 63.5:18:14:4.5.

Accordingly, the gel nail polish mixture is a dryable mixture being dried under an exposure of LED light, UV light, or sunlight.

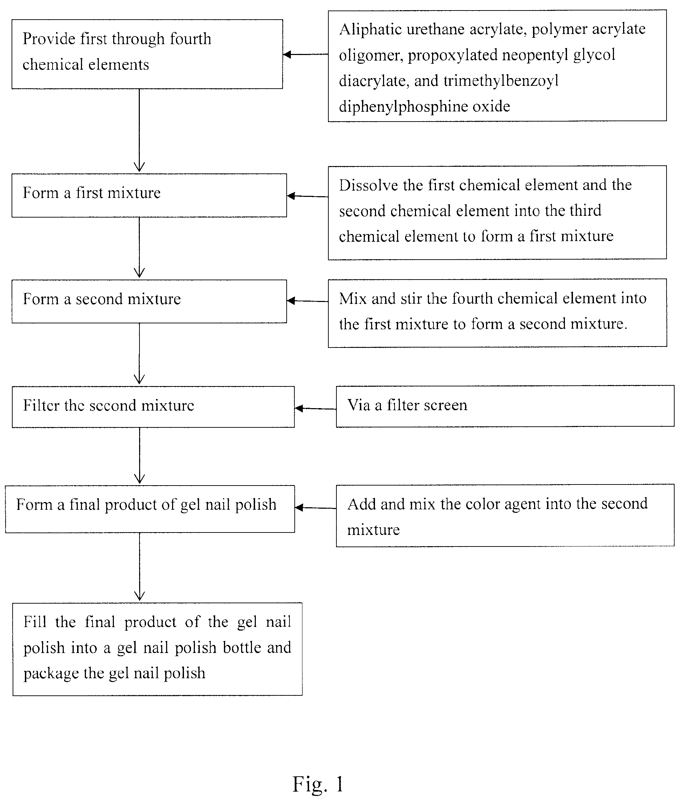

Referring to Fig. 1 of the drawings, the present invention provides a method of manufacturing the gel nail polish, wherein the manufacturing method comprises the following steps:
(1) Provide first through fourth chemical elements. Accordingly, the first through fourth chemical element and their weight ratio are described above.
(2) Dissolve the first chemical element and the second chemical element into the third chemical element to form a first mixture. Accordingly, the first chemical element and the second chemical element are stirred into the third chemical element for a predetermined period of time under a predetermined temperature until the first chemical element and the second chemical element are well mixed into the third chemical element. According to the present embodiment, the first chemical element and the second chemical element are preferably stirred into the third chemical element for 40 minutes under 60°C in order to ensure the first chemical element and the second chemical element to be well-mixed into the third chemical element. In other words, the first and second chemical element are gradually heated up from room temperature to 60°C when the first and second chemical element are added and disslove into the thrid chemical element.
(3) Mix and stir the fourth chemical element into the first mixture to form a second mixture. In addition, the second mixture is stirred for a predetermined period of time under room temperature until the fourth chemical element is well-mixed into the first mixture. According to the present embodiment, the second mixture is preferably stirred for 30 minutes under room temperature to ensure the fourth chemical element is well-mixed into the first mixture. It is worth mentioning that after the fourth chemical element is dissolved in the first mixture, the second mixture should be cooled down from 60°C back to the room temperature.
(4) Add and mix the color agent into the second mixture to form a final product of the gel nail polish. Accordingly, the color agent contains color pigments to form the final product of the gel nail polish with a desired color.
(5) Fill the final product of the gel nail polish into a gel nail polish bottle and package the gel nail polish bottle with the gel nail polish therein, such that the gel nail polish is ready to use.

According to the preferred embodiment, before the step (4), the manufacturing method further comprises a step of filtering the second mixture to remove residues in the second mixture before the color agent is added thereinto. Accordingly, the second mixture is filtered by screen, such as a 200 mesh gauze screen, to ensure the standardized particle size of the third mixture.

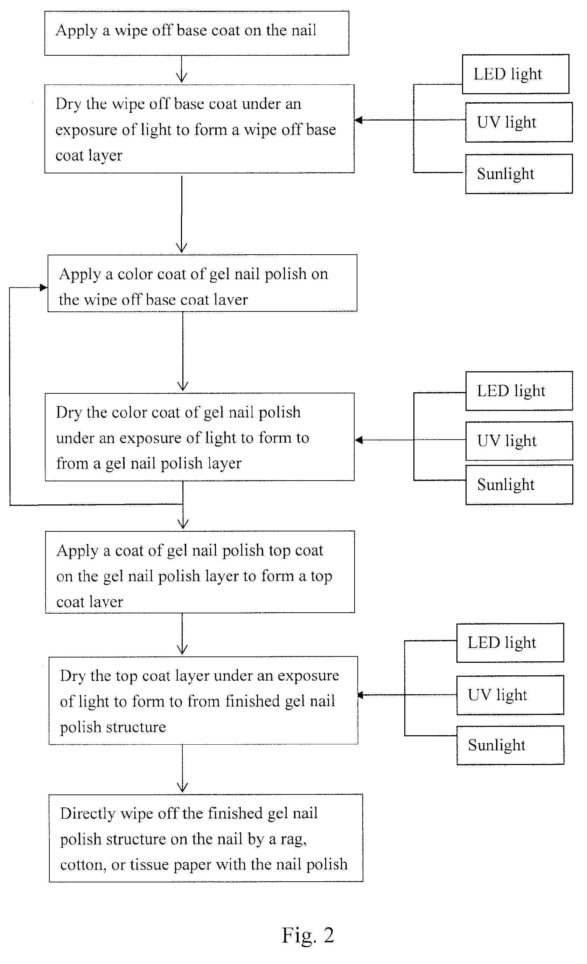

Referring to Fig. 2 of the drawings, the present invention further provides a method of using the gel nail polish, wherein the using method comprises the following steps:
(A) Apply a wipe off base coat on the nail.
(B) Dry the wipe off base coat under an exposure of light to from a wipe off base coat layer.
(C) Apply a color coat of gel nail polish on the wipe off base coat layer.
(D) Dry the color coat of gel nail polish to form a gel nail polish layer.
(E) Apply a coat of gel nail polish top coat on the gel nail polish layer to form a top coat layer.
(F) Dry the gel nail polish top coat to form a finished gel nail polish structure, wherein when the gel nail polish top coat is dried, no wet sticky colloid is formed on the finished gel nail polish structure.
   The wipe off base coat layer, the gel nail polish layer and the top coat layer are completely dried, for example, under an exposure of LED light for 30 to 60 seconds, under an exposure of UV light for 2 minutes, or under an exposure of sunlight for 1 to 2 minutes. It is worth mentioning that there is no wet sticky colloid formed on the finished gel nail polish structure, such that the user does not need to use alcohol to remove wet sticky colloids on the finished gel nail polish structure formed by the conventional soak off gel nail polish.
   If necessary, repeat the steps (D) and (E) to apply a second color coat of gel nail polish on the nail. The second coat is applied on the first gel nail polish layer after the first gel nail polish layer is dried. The second coat is dried under an exposure of light to integrally form the second layer on the first gel nail polish layer as one single layer structure. It is worth mentioning that the finished gel nail polish structure is three-layered or four-layered gel nail polish, which is determined by the number of color gel nail polish layer applied on the nails.
(G) When removing the gel nail polish layer, the user may simply directly wipe off the finished gel nail polish structure by a rag, cotton, or tissue paper soaked with the nail polish remover. It is worth mentioning that the finished gel nail polish structure can be kept on the nail for as long as 7 to 14 days as it is originally applied thereon.

According to the preferred embodiment, the gel nail polish is able to be directly wiped off from the nail without using the aluminum foil to wrap around the fingertips with the cotton balls saturated with the nail polish remover, so that the user can prevent spending lots of time to wait until a gap generated between the nails and the finished gel nail polish structure. And, the user doesn't need to use cuticle pusher or iron pusher to pop the gel nail polish layer off the nail, so as to prevent the nails being uneven and not smooth. In addition, the user also doesn't need to nail file or nail buffer to completely remove the residue gel from the nail, so as to prevent the nails being thinner. In other words, the removing process of the gel nail polish of the present invention can dramatically reduce the damage happened on the nails as by the conventional way.

It is worth mentioning that the conventional gel nail polish cannot be dried by simply exposing under the natural sunlight. And, after the top coat layer is dried, some wet sticky colloids will residue on the layer, so that the users must use alcohol to remove the wet sticky colloids on the gel nail polish layer. Therefore, the gel nail polish of the present invention allows the user to save much time and money to apply and remove the gel nail polish on their nails.

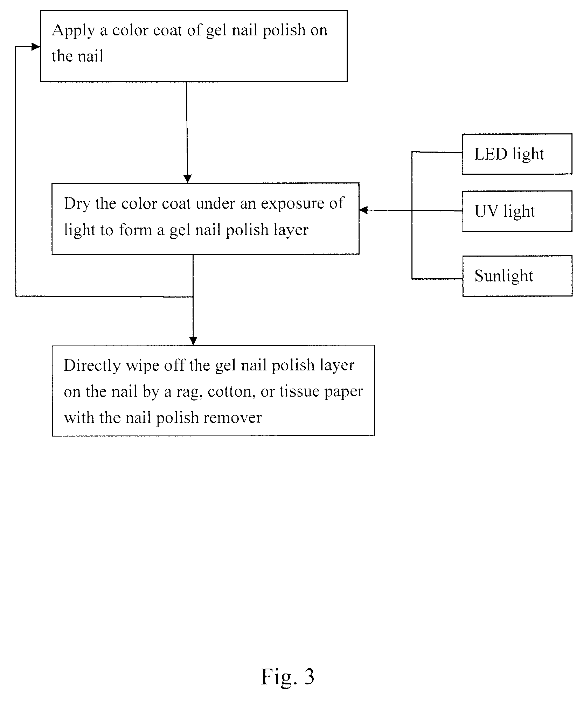

Referring to Fig. 3 of the drawings, the present invention further provides an alternative mode of a method of using the gel nail polish, wherein the using method comprises the following steps:
(A) Apply a color coat of gel nail polish on the nail. The user is able to apply a color coat of gel nail polish from the gel nail polish bottle via a conventional method. It is worth mentioning that no base coat and top coat is required before and after the gel nail polish layer is formed on the nail.
(B) Dry the color coat of the gel nail polish under an exposure of light to form the gel nail polish layer, wherein when the gel nail polish layer is dried, no wet sticky colloid is formed on the gel nail polish layer. The gel nail polish layer is completely dried, for example, under an exposure of LED light for 30 to 60 seconds, under an exposure of UV light for 2 mintues, or under an exposure of sunlight for 1 to 2 mintues. It is worth mentioning that there is no no wet sticky colloid formed on the gel nail polish layer, such that the user does not need to use alcohol to remove wet sticky colloids on the gel nail polish layer formed by the conventional nail polish.
   If necessary, repeat the steps (A) and (B) to apply a second color coat of gel nail polish on the nail. The second color coat is applied on the first gel nail polish layer after the first gel nail polish layer is dried. The second coat is also dried under an exposure of light to integrally form the second layer on the first gel nail polish layer as one single layer structure. The double layered gel nail polish is opaque and makes the color more intense and even on the nail.
(C) To remove the gel nail polish layer, directly wipe off the gel nail polish layer by a rag, cotton, or tissue paper with the nail polish remover. It is worth mentioning that the gel nail polish layer can be kept on the nail for 7 to 14 days as new as it is originally applied thereon.

One skilled in the art will understand that the embodiment of the present invention as shown in the drawings and described above is exemplary only and not intended to be limiting.

It will thus be seen that the objects of the present invention have been fully and effectively accomplished. The embodiments have been shown and described for the purposes of illustrating the functional and structural principles of the present invention and is subject to change without departure from such principles. Therefore, this invention includes all modifications encompassed within the spirit and scope of the following claims.

## Claims

1. A gel nail polish composition, comprising:
a gel nail polish mixture comprising an aliphatic urethane acrylate as a first chemical element, a polymer acrylate oligomer as a second chemical element, a monomer diluting agent as a third chemical element, and a photo initiator as a fourth chemical element, and
a color agent mixing with said gel nail polish such that said gel nail polish mixture is capable of being dried under an exposure of a light structure selected from the group consisting of an exposure of LED light, UV light and sunlight.

2. The gel nail polish composition, as recited in claim 1, wherein an amount of said first chemical element has a range of 57.6-69.9% by weight, an amount of said second chemcial element has a range of 16.4-19.8% by weight, an amount of said third chemcial element has a range of 12.7-15.4% by weight, and an amount of said fourth chemcial element has a range of 4.1-4.9% by weight.

3. The gel nail polish composition, as recited in claim 1, whrerin a weight ratio of said first through fourth chemcial element is 63.5:18:14:4.5.

4. The gel nail polish composition, as recited in claim 1, wherein said monomer diluting agent is a polymer acrylate oligomer and said photo initiator is a propoxylated neopentyl glycol diacrylate, and trimethylbenzoyl diphenylphosphine oxide.

5. The gel nail polish composition, as recited in claim 2, wherein said monomer diluting agent is a polymer acrylate oligomer and said photo initiator is a propoxylated neopentyl glycol diacrylate, and trimethylbenzoyl diphenylphosphine oxide.

6. The gel nail polish composition, as recited in claim 3, wherein said monomer diluting agent is a polymer acrylate oligomer and said photo initiator is a propoxylated neopentyl glycol diacrylate, and trimethylbenzoyl diphenylphosphine oxide.

7. A method of manufacturing a gel nail polish, comprising the steps of:
(a) providing an aliphatic urethane acrylate as a first chemical element, a polymer acrylate oligomer as a second chemical element, a monomer diluting agent as a third chemical element, and a photo initiator as a fourth chemical element;
(b) dissolving said first chemical element and said second chemical element into said third chemical element by heating and stirring said first and second chemical elements in said third chemical element to form a first mixture;
(c) mixing and stirring said fourth chemical element with said first mixture together to form a second mixture; and
(d) adding and mixing a color agent into said second mixture to form a final product of the gel nail polish.

8. The method, as recited in claim 7, wherein an amount of said first chemical element has a range of 57.6-69.9% by weight, an amount of said second chemcial element has a range of 16.4-19.8% by weight, an amount of said third chemcial element has a range of 12.7-15.4% by weight, and an amount of said fourth chemcial element has a range of 4.1-4.9% by weight.

9. The method, as recited in claim 8, wherein a weight ratio of said first through fourth chemcial element is 63.5:18:14:4.5, wherein said monomer diluting agent is a polymer acrylate oligomer and said photo initiator is a propoxylated neopentyl glycol diacrylate, and trimethylbenzoyl diphenylphosphine oxide.

10. The method, as recited in claim 9, wherein in the step (b), said first mixture is stirred for 40 minutes under 60°C to ensure siad first and second chemical elements to be dissolved in said third chemical element, wherein in the step (c), said fourth chemical element and said first mixture are stirred for 30 minutes under room temperature to form said second mixture.

11. The mentod, as recited in claim 7, before the step (c), further comprising a step of filtering said second mixture to remove residues in said second mixture before said color agent is added thereinto.

12. A method for using a gel nail polish, comprising the steps of:
(a) applying a color coat of gel nail polish on a nail, wherein said gel nail polish comprises an aliphatic urethane acrylate as a first chemical element, a polymer acrylate oligomer as a second chemical element, a monomer diluting agent as a third chemical element, and a photo initiator as a fourth chemical element;
(b) drying said color coat of gel nail polish under an exposure of a light structure selected from the group consisting of LED light, UV light and sunlight for two minutes or less to form a gel nail polish layer; and
(c) directly wiping off said gel nail polish layer by a rag, cotton, or tissue paper with the nail polish remover when removing said gel nail polish layer.

13. The method, as recited in claim 12, wherein an amount of said first chemical element has a range of 57.6-69.9% by weight, an amount of said second chemcial element has a range of 16.4-19.8% by weight, an amount of said third chemcial element has a range of 12.7-15.4% by weight, and an amount of said fourth chemcial element has a range of 4.1-4.9% by weight.

14. The method, as recited in claim 13, wherein a weight ratio of said first through fourth chemcial element is 63.5:18:14:4.5, wherein said monomer diluting agent is a polymer acrylate oligomer and said photo initiator is a propoxylated neopentyl glycol diacrylate, and trimethylbenzoyl diphenylphosphine oxide.

15. The method, as recited in claim 12, wherein, after the step (b), further comprises a step of repeating the steps (a) and (b), for applying a second color coat of gel nail polish on the nail to form a double-layeyed gel nail polish layer
